# EUROPÄISCHE PATENTANMELDUNG

(11) **EP 2 181 698 A2**
(43) Veröffentlichungstag der Anmeldung: **05.05.2010**
(21) Anmeldenummer: 09013343.0
(22) Anmeldetag: 22.10.2009
(51) Int. Cl.: A61K 8/891, A61K 8/895, A61K 8/29, A61K 8/49, A61K 8/19, A61K 8/25, A61K 8/44, A61Q 17/00, A61Q 19/00

(54) **Kosmetische Formulierung mit Siloxanelastomeren und partikulären Stoffen**

(30) Priorität: 22.10.2008 DE 102008053791
(71) Anmelder: Beiersdorf AG, 20245 Hamburg (DE)
(72) Erfinder: Storbeck, Celina, 25474 Bönningstedt (DE); Skubsch, Kerstin, 25497 Prisdorf (DE); Köhler, Manuela, Dr., 20144 Hamburg (DE); Reidel, Heidi, 22083 Hamburg (DE); Münchow, Lynn, 22547 Hamburg (DE); Miertsch, Heike, 22459 Hamburg (DE)
(74) Vertreter: Hartmann, Jost

(57) **Zusammenfassung**

Die Erfindung beschreibt kosmetische Zubereitungen umfassend ABC-Siloxanelastomere in Kombination mit partikulären Stoffen.
Die Verwendung von ein oder mehreren ABC-Siloxanelastomere zur verbesserten Dispergierbarkeit und/oder zu einem verbessertem Sedimentationsverhalten partikulärer Stoffe in kosmetischen Formulierungen sowie die Verwendung von ein oder mehreren ABC-Siloxanelastomere in kosmetischen Sonnenschutzformulierungen mit einem Gehalt an partikulären UV-Filtern zur Steigerung des UV Schutzes der Formulierung, zur Verbesserung des sensorischen Hautgefühls der Formulierung und/oder zu Erhöhung der Viskosität der Formulierung wird auch beansprucht.

## Beschreibung

Die Erfindung beschreibt kosmetische Zubereitungen umfassend ABC-Siloxanelastomere in Kombination mit partikulären Stoffen.

Siloxanelastomere werden in kosmetischen Zubereitungen aufgrund ihrer seidigen und pudrigen Sensorikeigenschaften eingesetzt.

US 5628989 beschreibt Formulierungen mit sogenannten "Silicone Rubber Powder", die ein angenehmes Hautgefühl verursachen. Nachteilig ist, dass andere Bestandteile der Formulierungen, insbesondere partikuläre Bestandteile, schwierig einzuarbeiten sind und ansonsten deren Partikelgröße exakt eingehalten werden muss.

US 5922308 beschreibt Elastomere, sogenannte "crosslinked non-emulsifying siloxane elastomer", in Kombination mit flüchtigem Silikonöl zur Verhinderung von Synärese bei wasserfreien Antitranspirant-Cremes.

EP 909162 beschreibt Silikongele aus einem flüchtigem Silikonöl und einem vernetztem Organopolysiloxanmaterial.

EP 1542652 beschreibt quaternierte aminofunktionale Siloxanelastomere in flüchtigem oder nicht flüchtigem Silikonöl.

Nachteil bei all diesen Elastomeren ist deren Einschränkung auf Silikonöle bzw. auf zwingend flüchtige Silikonöle und dass die Verarbeitung in Kosmetika nur mit flüchtigem Silikonöl funktioniert.

DE 102005019548, WO 2005094775 und EP 1391193 beschreiben bekannte Siloxanelastomere wie Dimethicon/Vinyl Dimethicon Crosspolymer oder Dimethicon/Polysilicon 11.

In den WO 2007109260, WO 2007109240 und WO 2008085360 werden neuartige Siloxanelastomere beschrieben. Diese Silioxanelastomere, genauer Siloxanpolyetherelastomere, erhält man durch die Reaktion eines Organosiloxans mit einem, mindestens zwei SiH Gruppen enthaltendem Cyclosiloxan und einer Substanz oder mehreren Substanzen, mit mindestens 2 aliphatischen ungesättigten Gruppen im Molekül unter Verwendung eines Hydrosilylierungskatalysators. Diese in den WO 2007109260, WO 2007109240 und WO 2008085360 beschriebenen Siloxanelastomere in Gelform können mit Körperpflege- oder Gesundheitsstoffen, die pharmakologische Wirkungen zeigen, wie Vitamine oder Hormone, kombiniert werden.

Bedeutsam wäre es aber darüber hinaus für den kosmetischen Einsatz stabile und vielfältig einsetzbare kosmetische Zubereitungen mit den neuartigen Silixoanelastomeren bereit zu stellen. Insbesondere ist die Aufgabe stabile kosmetische Zubereitung bereit zu stellen, die ein oder mehrere partikuläre Stoffe enthalten.
Aufgabe ist es eine alternative kosmetische Zubereitung mit verbesserter Stabilität bereit zu stellen.

Gelöst werden diese Aufgaben durch kosmetische Formulierungen umfassend ABC-Siloxanelastomere in Kombination mit partikulären Stoffen entsprechend Anspruch 1.

Die erfindungsgemäße kosmetische Formulierung umfasst ein oder mehrere ABC-Siloxanelastomere, erhältlich aus der Reaktion von
A - Organohydrogensiloxanen mit mindestens zwei SiH tragenden Cyclosiloxanringen pro Molekül mit
B - Stoffen oder Mischungen mit mindestens zwei aliphatischen ungesättigten Gruppen oder Hydrocarbongruppen pro Molekül und einem
C - Hydrosilylationskatalysator,
in Kombination mit einem oder mehreren partikulären Stoffen und ggf. polaren Ölen und/oder Wachse.

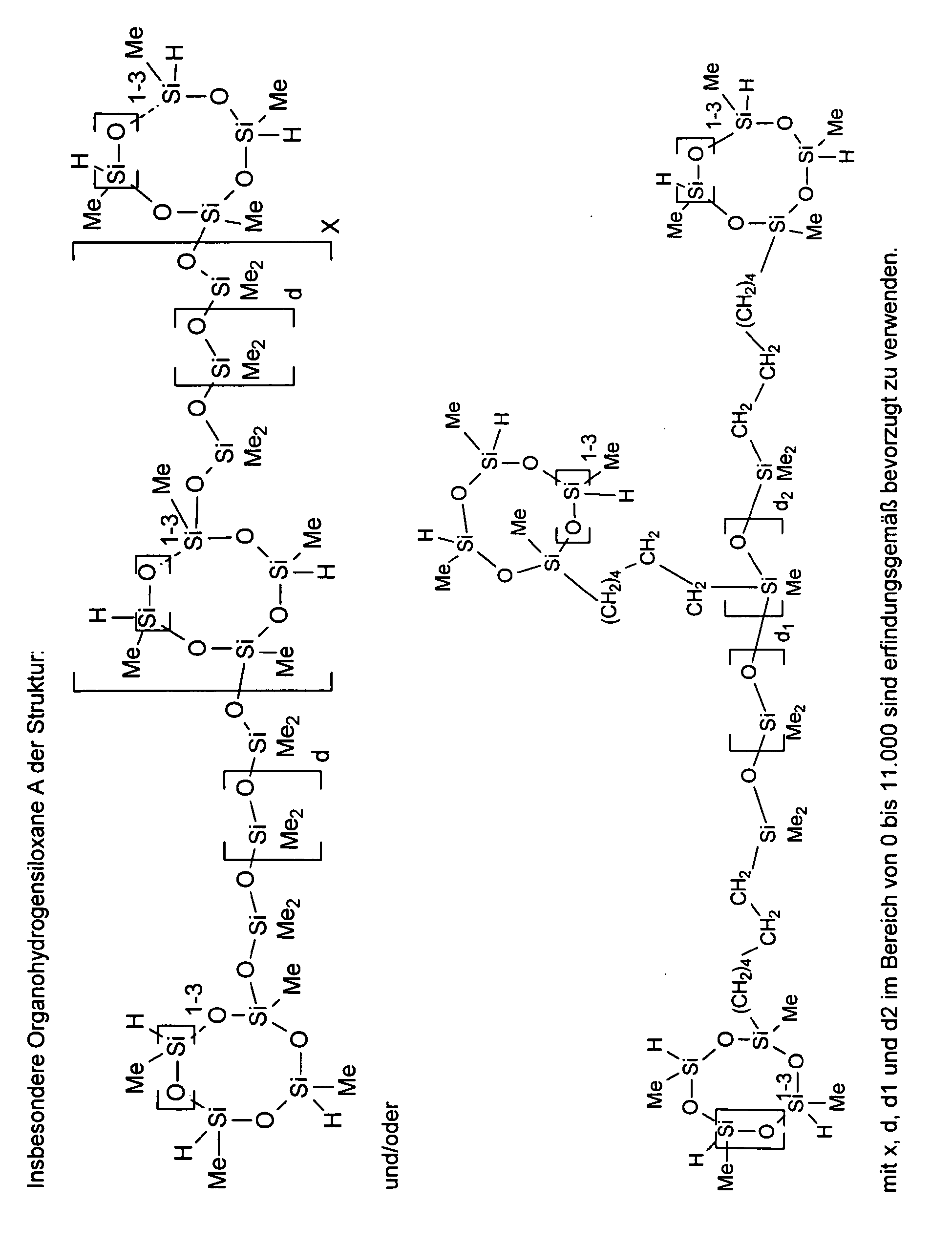

Erfindungsgemäße ABC-Siloxanelastomere sind vor allem die in den Druckschriften WO 2007109260, WO 2007109240 und WO 2008085360 beschriebenen Silikon Elastomere, Silikon Polyether Elastomere und deren Gelformen. Es wird hiermit ausdrücklich zu den in der Druckschriften WO 2007109260, WO 2007109240 und WO 2008/085360 dargestellten Elastomeren und deren Herstellung Bezug genommen.
Bevorzugte Beispiele der erfindungsgemäßen Siloxanelastomere sind Dimethicone/PPG-20 Crosspolymer, Dimethicone/ Bis-Isobutyl PPG-20 Crosspolymer bzw. Bis-Vinyl Dimethicone/PPG-20 Crosspolymer.

Diese erfindungsgemäßen neuartigen ABC-Siloxanelastomere unterscheiden sich von den klassischen bekannten Siloxanelastomeren, wie das DimethiconNinyl Dimethicon Crosspolymer, aufgrund einer unterschiedlichen chemischen Struktur und bedingen dadurch auch Unterschiede in ihren Eigenschaften.

Die Probleme der Einarbeitung partikulärer Stoffe und anderer als Silikonöle bzw. flüchtige Silikonöle mit Siloxanelastomeren in kosmetischen Zubereitungen war bekannt. Erstaunlich ist, dass sich nun erfindungsgemäß eine Kombination aus partikulären Stoffen und den ABC-Siloxanelastomeren stabil in kosmetische Zubereitungen einarbeiten lassen und die Einarbeitung auch ohne flüchtiges Silikonöl funktioniert. Ein Verzicht auf flüchtige Silikonöle ist dabei möglich und bevorzugt. Die ABC-Siloxanelastomere allein ließen noch keine Verbesserung der Stabilität kosmetischer Zubereitungen erwarten.

Bevorzugte Anteile der ABC-Siloxanelastomere liegen im Bereich von 1 bis 25 Gew.%, bezogen auf die Gesamtmasse der Zubereitung, insbesondere im Bereich von 2 bis 10 Gew.%.

Die erfindungsgemäßen ABC-Siloxanelastomere entsprechend der WO 2007109260 und WO 2007109240 weisen im Gegensatz zu herkömmlichen Elastomeren den überraschenden Unterschied auf, dass sie nicht nur mit leicht flüchtigen Silikonölen kompatibel sind, sondern auch mit vielen anderen in kosmetischen und pharmazeutischen Formulierungen verwendeten natürlichen und synthetischen, nicht ausschließlich polaren, Ölen kompatibel sind.

Aufgrund der Kombination mit partikulären Stoffen und gegebenfalls polaren Ölen oder Wachsen ergeben sich verschiedene überraschende Möglichkeiten, die im Folgenden beschrieben werden sollen:

Die kosmetische Zubereitungen umfassend diese Kombination
- weisen eine bessere Dispergierbarkeit partikulärer Bestandteile, wie zum Beispiel UV Filter, Pigmente, Wirkstoffe und Füllstoffe, auf,
- zeigen eine erhöhte Farbintensität und Deckkraft,
- haben eine bessere Stabilität und ermöglichen die Verhinderung der Auskristallisation oder Synärese durch eine verbesserte Wachskompatibilität,
- zeigen eine erhöhte Wasserfestigkeit.

Somit lässt sich erstaunlicherweise aufgrund der ABC-Siloxanelastomere eine verbesserte Dispergierbarkeit von partikulären Bestandteilen, aus denen die erfindungsgemäßen partikulären Stoffe gewählt werden können, wie
1. UV-Filter
2. farbgebende Pigmente, wie vorzugsweise TiO2, Eisenoxide oder organische Farblacke
3. Wirkstoffe, wie insbesondere Deodorantien oder Antitranspirantien
4. Füllstoffe
in kosmetischen Formulierungen erreichen.

Als partikuläre Stoffe sind erfindungsgemäß besonders bevorzugt UV-Filter, farbgebende Pigmente, wie vorzugsweise TiO2, Eisenoxide oder organische Farblacke, Wirkstoffe, wie insbesondere Deodorantien oder Antitranspirantien, und/oder Füllstoffe zu wählen.

Es haben sich folgende überraschende Effekte ergeben:

### 1. UV-Filter:

Die Kombination der ABC-Siloxanelastomere mit partikulären UV-Filtern in kosmetischen Zubereitungen führt zu einem erhöhten Lichtschutzfaktor. Zur Bestimmung der Wirksamkeit von kosmetischen Sonnenschutzprodukten vor UVB-Strahlung wird die seit 1994 leicht abgeänderte COLIPA-Methode (frz. Comité de Liaison des Associations Européennes de industrie de la Parfumerie, des Produits Cosméthiques et de Toilette »Verbandsausschuss der europäischen Vereinigung der Parfüm-, Kosmetik- und Toilettenartikelindustrie«), d.h. die "Internationale Methode zur Bestimmung des Lichtschutzfaktors", einheitlich in Europa angewendet. Bei dieser "in-vivo"-Testmethode wird der Lichtschutzfaktor bestimmt. Dieser gibt an, um welchen Faktor die Dauer der Sonnenexposition der jeweiligen Hauttypen verlängert werden kann, ohne dass sich ein Erythem der Haut bildet.

Vorteil der erfindungsgemäßen Zubereitungen ist zudem, dass auch die UVA-Schutzleistung verbessert wird (UVA-Boost), sowohl ohne Vorbestrahlung nach der UVA-Balance Methode (nach DIN 67502) als auch nach Vorbestrahlung gemäß der neuen Colipa Ratio Methode.

Bei partikulären Lichtfiltern stellt sich ein verbessertes sensorisches Hautgefühl ein, d.h. erfindungsgemäße Zubereitungen mit ABC-Siloxanelastomeren und partikulären Lichtfiltern sind weniger stumpf und trocken und zeigen geringeres Weißeln.

Sonnenschutzzubereitungen werden sehr häufig deutlich dünnflüssiger, wenn oberflächenaktive Pigmente wie bestimmte Titandioxide, z.B. Uvinul T 805 oder wie in DE102006013283 beschriebene, oder organische Lichtfilterpigmente, wie 2,4,6-Tris-(biphenyl)-1,3,5-Triazin, (2-{4-[2-(4-Diethylamino-2-Hydroxy-Benzoyl)-Benzoyl]-Piperazine-1-Carbonyl}-Phenyl)-(4-Diethylamino-2-Hydroxy-Phenyl)-Methanon oder 2'-Methylen-bis-(6-(2H-benzotriazol-2-yl)-4-(1,1,3,3-tetramethylbutyl)-phenol), enthalten sind. Einige dieser Pigmente sind außerdem nicht mit allen Verdickern kompatibel, welche im Allgemeinen verwendet werden, um diesen Phänomen entgegen zu wirken. Erstaunlicherweise führen diese Pigmente in Kombination mit den ABC-Siloxanelastomeren zu einer höheren Viskosität der sie enthaltenen Zubereitung, im Vergleich zu den elastomerfreien Formulierungen. Durch Zugabe des ABC-Siloxanelastomere können somit höhere Viskositäten erzielt werden, so dass das Produkt beispielsweise nicht aus der Flasche läuft.

Als partikuläre UV-Filter werden bevorzugt eingesetzt:
2'-Methylen-bis-(6-(2H-benzotriazol-2-yl)-4-(1,1,3,3-tetramethylbutyl)-phenol); Titandioxid, Zinkoxid, 2,4,6-Tris-(biphenyl)-1,3,5-triazin, (2-{4-[2-(4-Diethylamino-2-Hydroxy-Benzoyl)-Benzoyl]-Piperazine-1-Carbonyl}-Phenyl)-(4-Diethylamino-2-Hydroxy-Phenyl)-Methanon.

Zudem ist es von Vorteil wenn weitere nicht partikuläre UV-Filter enthalten sind, gewählt aus der Gruppe: Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2; 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); Merocyanine

Als erfindungsgemäße partikuläre Stoffe können auch farbgebende Pigmente gewählt werden.

### 2. Farbgebende Pigmente

Üblicherweise werden durch klassische Elastomere stark mattierende Effekte, wie z.B. in den WO 1999022696 A1, US 6027738 A1 und EP 975309 B1 beschrieben, erreicht. Durch die verbesserte Dispergierbarkeit der farbgebenden Pigmente durch die hydrophile Gruppe der ABC-Siloxanelastomere werden erstmalig intensive lebendige Farbeffekte, eine erhöhte Farbintensität und eine erhöhte Deckkraft erreicht.

Die Kombination ABC- Siloxanelastomer und farbgebendes Pigment ermöglicht erstmals diese Verbesserungen in kosmetischen Zubereitungen.

### 3. Auch partikuläre Wirkstoffe können als partikuläre Stoffe gewählt werden.

Aufgrund der verbesserten Dispergierbarkeit lässt sich auch eine verbesserte Freisetzung von beispielsweise partikulären Deo- oder Antitranspirant (AT)-Wirkstoffen erreichen, die somit effizienter eingesetzt werden können

### 4. Füllstoffe

Partikuläre Füllstoffe zeigen mit den ABC-Siloxanelastomeren ein verbessertes Sedimentationsverhalten. Dies ist insbesondere auch für die unter 3. erwähnten AT-Wirkstoffe vorteilhaft.
In wasserfreien Systeme werden die AT-Wirkstoffe häufig in Pulverform eingesetzt. Um eine gute Wirksamkeit zu erzielen, dürfen die AT-Wirkstoffe nicht zu schnell sedimentieren. Das schnelle Sedimentieren war in der Vergangenheit jedoch häufig ein Problem.
Erfindungsgemäß wird dies nun durch die Kombination mit den ABC-Siloxanelastomeren gelöst. Das Elastomer erfüllt dabei nun zwei Funktionen:
a. verbessertes Hautgefühl und
b. als Suspendierhilfe.
Somit kann auf klassische Suspendierhilfen wie Schichtsilikate oder Kieselsäuren bei den erfindungsgemäßen Zubereitungen verzichtet werden.

### 5. - 6. Dekorative Füllstoffe:

Die erfindungsgemäßen Zubereitungen können ferner Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Auch diese Füllstoffe zeigen mit den ABC-Siloxanelastomeren ein verbessertes Sedimentationsverhalten. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Cellulosen und Cellulosederivate, Pigmente, die weder hauptsächlich UV-Filter- noch färbende Wirkung haben (wie z. B. Bornitrid etc.) sowie Lauroyl Lysine, Polymethylsilesquioxane, Polymethylmethacrylate, Polymethylmethacrylate Crosspolymer, Polyamide, Nylon, Polyethylen-Pulver, Talkum, beschichtetes Talkum, z.B. mit Dimethicone und Trimethylsiloxysilicate, Mica, Silica.

Neben den partikulären Stoffen sind auch polare Öle oder Wachse in Kombination mit ABC-Siloxanelastomeren zur Stabilitätsverbesserung kosmetischer Zubereitungen zu verwenden.

So zeigen die ABC-Siloxanelastomere mit polaren Ölen, also insbesondere anderen Ölen als Silikonölen, eine verbesserte Kompatibilität und ermöglichen so eine vielfältigere Sensorik, bessere Stabilisierung und Freisetzung von Wirkstoffen (1.-4.).

Aus der verbesserten Kompatibilität mit verschiedenen, auch polaren Ölen im Gegensatz zu herkömmlichen Siloxanelastomeren kann erfindungsgemäß auf unpolare und insbesondere flüchtige Öle (Kohlenwasserstoffe, Silikonöle) in kosmetischen Formeln zukünftig verzichtet bzw. deren Menge reduziert werden, was einen erheblichen Formulierungsvorteil und -freiraum bietet.
Auf die in kosmetischen Zubereitungen umfassend Siloxanelastomeren ansonsten enthaltenen Silikonöle, insbesondere flüchtige Silikonöle kann erfindungsgemäß verzichtet werden. Vorteilhaft sind die erfindungsgemäßen Zubereitungen daher silikonölfrei. Frei hießt dabei ein Anteil von weniger als 1 Gew.%, bezogen auf die Gesamtmasse der Zubereitung, bevorzugt weniger als 0,1 Gew.% an Silikonölen.

Öle und Fette unterscheiden sich unter anderem in ihrer Polarität. Es wurde vorgeschlagen, die Grenzflächenspannung gegenüber Wasser als Maß für den Polaritätsindex eines Öls bzw. einer Ölphase anzunehmen. Dabei gilt, dass die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar werden Lipide angesehen, deren Grenzflächenspannung gegen Wasser weniger als 20 mN/m beträgt, als unpolar solche, deren Grenzflächenspannung gegen Wasser mehr als 30 mN/m beträgt. Lipide mit einer Grenzflächenspannung gegen Wasser zwischen 20 und 30 mN/m werden im Allgemeinen als mittelpolar bezeichnet.

Erfindungsgemäß ist es nun möglich Lipide mit einer Grenzflächenspannung kleiner 30 mN/m in Kombination mit den ABC-Siloxanelastomeren in kosmetischen Zubereitungen einzusetzen.

Auf den Zusatz unpolarer Lipide mit einer Grenzflächenspannung größer 30 mN/m kann hingegen verzichtet werden bzw. deren Gehalt erheblich reduziert werden.

Beispielsweise sind polare Öle, wozu auch flüssige UV-Filter zählen, notwendig um eine ausreichende Menge schwerlöslicher UV-Filter zu lösen, die wiederum nötig sind, um höhere Lichtschutzleistungen zu erreichen.

Der Verbraucher bevorzugt immer höhere Lichtschutzleistungen, um sich vor der Sonne zu schützen, möchte aber auf ein angenehmes Hautgefühl beim Auftragen solcher Formulierungen nicht verzichten. Herkömmliche Siloxanelastomere sind normalerweise nicht mit polaren Ölen kompatibel, beeinflussen allerdings das Hautgefühl positiv. Mit den ABC-Siloxanelastomeren ist es erstmals möglich, hohe Mengen an ansonsten schwerlöslichen UV-Filtern einzuarbeiten und nicht auf ein angenehmes Hautgefühl zu verzichten. Die Löslichkeit der schwerlöslichen UV-Filter ist erhöht, wenn das erfindungsgemäße ABC-Siloxanelastomer eingesetzt wird.
Somit ist auch erstmals erhöhter Lichtschutz (SPF) und gleichzeitig erhöhter UV-A Schutz mit den erfindungsgemäßen Formulierungen möglich.

Zusätzlich wird durch die neuartigen Elastomere ein zusätzlicher SPF und UV-A Boost gewährleistet, d.h. die Lichtschutzleistung wird insgesamt erhöht gegenüber Formeln, die das neuartige Elastomer nicht enthalten.

Vorteilhafte polare Ölkomponenten können im Sinne der vorliegenden Erfindung gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können vorteilhaft gewählt werden aus der Gruppe Phenethylbenzoat, 2-Phenylethylbenzoat, Propylheptyl Caprylate, Isopropyl Lauroyl Sarkosinat, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.
Ferner können vorteilhaft gewählt werden Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche. Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht. Weitere Ölkomponenten können sein: Caprylic/Capric Triglyceride, Octyldodecanol, C12-15 Alkyl Benzoate, Butylenglycol Dicaprylat/Dicaprat, Dicaprylyl Carbonat, Isopropyl Palmitate, Ethylhexyl Cocoate, Cera Microcristallina + Paraffinum Liquidum, Butyrospermum Parkii, Dicaprylyl Ether, Hydrogenated Coco-Glycerides, Simmondsia Chinensis Oil, Tridecyl Stearate, Tridecyl Trimellitate, Dipentaerythrityl Hexacaprylate/Hexacaprate, Lanolin Alkohol.

Insbesondere Vorteilhaft sind Lipide mit einer Grenzflächenspannung gegen Wasser im Bereich von 20 bis 29,9 mN/m. Insbesondere werden die polaren Lipide gewählt aus der Gruppe C₁₂₋₁₅-Alkylbenzoat; Butylenglycol Dicaprylat/Dicaprat, Dicaprylyl Carbonat, Isodecyl Neopentanoate und Caprylic/Capric Triglyceride,

Ebenfalls ist die Flüchtigkeit der Öle ein Charakteristikum zu dessen Einsatzmöglichkeit. Erfindungsgemäß kann auf den Zusatz bei Raumtemperatur flüchtigen Ölen ebenso verzichtet werden bzw. deren Gehalt vermindert werden.

Weiterer erfindungsgemäßer Vorteil, der aus der Einarbeitung der ABC-Siloxanelastomere in kosmetische Formulierungen resultiert, ist eine Verbesserung der Wasserfestigkeit der Zubereitungen.
Der Nachweis der verbesserten Wasserfestigkeit erfolgt mit Hilfe der in-vitro Wasserfestigkeitsmethode wie sie in DE 102007028497 beschrieben ist.

Dekorative Zubereitungen und/oder Deodorantien bzw. Antitranspirantien basieren häufig auf pastösen bis festen, wachshaltigen Zubereitungen. Sie sind mitunter wasserhaltig oder wasserfrei.

Erstaunlicherweise zeigt sich durch den Zusatz an ABC-Siloxanelastomeren eine verbesserte Kompatibilität mit Wachsen und die dekorativen Zubereitungen und/oder Deodorantien bzw. Antitranspirantien weisen eine vorteilhaft glatte, homogene Struktur auf und ermöglichen damit eine erhöhte und verbesserte Applikation/Pay-off. Die verbesserte Applikation und der hohe Pay-Off zeichnen sich bei den dekorativen Zubereitungen durch hohe Farbintensität nach einmaliger Applikation aus, sowie z.B. einem geschmeidig, gleitenden Lippengefühl während der Applikation. In dekorativen Zubereitungen wird hierdurch ein erhöhter Glanz erreicht.
Das Eintrocknen der pastösen Zubereitungen wird zudem vermieden, was insbesondere bei Mascara-Zubereitungen wünschenswert ist.

Bei Deodorantien bzw. Antitranspirantien ermöglichen die ABC Elastomer eine verbesserte Applikation und eine homogenere Verteilung der Wirkstoffe auf der Haut. Darüber hinaus kann durch die homogene Struktur und den gleichmäßigen Auftrag die Bildung von Rückständen auf der Haut und Kleidung minimiert werden, da die Formulierungen nur in einer dünnen Schicht auf die Haut aufziehen und dann auch sehr schnell einziehen können.

Die bessere Wachskompatibilität der ABC-Siloxanelastomere, insbesondere den polaren Wachse, führt bei Stiftformulierungen zu einer besseren Stabilität sowie einer Verhinderung der Auskristallisation und Synärese.

Dekorative Formulierungen wie Foundations umfassen W/S-Emulsionen und/oder wasserfreie Zubereitungen. Mascara stellen O/W-Emulsionen oder wasserfreie Zubereitungen dar und Lippenstifte sind wasserfreie Zubereitungen.
Foundations auf Basis einer W/S-Emulsion oder wasserfreien Zubereitung zeichnen sich aufgrund hoher Gehalte an flüchtigen Ölen (Silikonöle und Kohlenwasserstoffe) durch eine ausgezeichnete Haftfestigkeit auf der Haut und damit durch eine lange Haltbarkeit auf der Haut aus. Nachteilig sind diese Systeme aber darin, dass sie wenig pflegend wirken, da sie aufgrund hoher Anteile flüchtiger Komponenten, wie Silikonöle, kaum einen Pflegerückstand auf der Haut hinterlassen. Werden Pflegekomponenten wie polare, nicht flüchtige Öle und Wachse zugegeben, zeigen die Foundations starke Instabilitäten (Ölabscheidung).
Durch Zusatz der ABC-Siloxaneelastomere wird der Einsatz hautpflegender und rückfettender polarer Öle und Wachse erstmals ohne Stabilitätseinbußen möglich. O/W-Mascaras bedingen durch ein hohes Wachsaufnahmevermögen hervorragende Volumen- und längegebende Eigenschaften und verleihen den Augen eine hohe Ausdrucksstärke. Wasserfreie Mascara-Systeme liefern eine hohe Wasserresistenz und ein lange Haltbarkeit auf den Wimpern. Beide Systeme zeichnen sich durch eine pastöse Struktur aus und trocknen stark über die Zeit aus. Dies äußert sich in einer krümeligen Struktur bzw. in Krümeln auf den Wimpern.
Durch den Einsatz der ABC-Siloxaneelastomere wird dieses Eintrocknen vermieden.

Wasserfreie Lippenstifte weisen einen hohen Gehalt an natürlichen Ölen und Esterwachsen auf, die einen hohen Pflegecharakter vermitteln. Nachteilig ist, dass diese ein schmieriges, fettiges Lippengefühl erzeugen. Ein leichtes, seidig- glattes Lippengefühl von Lippenstiften und Glossen konnte bisher nicht bereitgestellt werden, da sowohl Silikonöle als auch klassische Siloxane Elastomere (z.B. im Handel erhältliche DC 9040, DC 9041, Dow Corning 9506) Inkompatibilitäten und Instabilitäten aufweisen. Durch den Einsatz der ABC-Siloxanelastomere ist die Kompatibilität überraschenderweise erstmals gewährleistet. Hierdurch lässt sich erstmals ein seidig, leichtes, glattes Lippengefühl in Lippenprodukten erzielen.

Durch Einsatz der ABC-Siloxanelastomere mit hydrophilen Gruppen werden Farbpigmente überraschenderweise hervorragend dispergiert und hierdurch die Farbintensität und die Deckkraft der dekorativen Zubereitungen deutlich erhöht.

Aufgrund der zuvor erläuterten Möglichkeit der Reduzierung oder des Verzichts auf flüchtige Silikonöle bedingt durch die Zugabe der ABC-Siloxanelastomere zeigt sich bei Deo- / AT-Zubereitungen ein verändertes Hautgefühl und eine Reduzierung weißer Rückstände. Die nun nutzbaren polaren Öle können die weißen Rückstände reduzieren und die sensorischen Eigenschaften positiv beeinflussen.
Des weiteren führt der Einsatz von polareren Ölen in Deo / AT-Zubereitungen zu einer besseren Vorbereitung der Haut für eine Achselhaarrasur.

Durch Einsatz der ABC-Siloxanelastomere können ansonsten instabile Formulierungen stabilisiert werden, bzw. bei stabilen Formulierungen kann die Emulgatormenge verringert werden.
Gleichzeitig sind die Formeln auch stabiler gegenüber dem Zusatz von Elektrolyten wie z.B. 2-Phenylbenzimidazol-5-sulfonsäuresalze .

Die ABC-Siloxanelastomere weisen demnach auch eine Emulsions stabilisierende Wirkung auf.

Liegt die erfindungsgemäße Zubereitung in Form einer O/W-Emulsion vor, so ist sie erfindungsgemäß bevorzugt **dadurch gekennzeichnet, dass** die Zubereitung einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , PEG-100 Stearat, Sucrose Polystearate in Kombination mit hydriertem Polyisobuten, Natriumstearoylglutamat, Natriumcetearylsulfat enthält. Ferner ist es vorteilhaft im Sinne der vorliegenden Erfindung Cetearylalkohol in Kombination mit PEG-40 hydriertes Rizinusöl, Natriumcetearylsulfat und Glycerylstearat zu wählen. Ausserdem ist es erfindungsgemäß vorteilhaft Kaliumcetylphosphat als Emulgator einzusetzen. Desweiteren ist es erfindungsgemäß vorteilhaft Sorbitan Stearate, Silikonpolyether Copolymere wie zum Beispiel PEG-10 Dimethicone, PEG/PPG-14/4 Dimethicone, Glyceryl Stearate Citrate und/oder Sodium Stearoyl Glutamate auszuwählen.

Die erfindungsgemäßen Zusammensetzungen können ferner gegebenenfalls in der Kosmetik übliche Zusatzstoffe, beispielsweise Parfüm, Verdicker, Desodorantien, antimikrobielle Stoffe, rückfettende Agentien, Komplexierungs- und Sequestrierungsagentien, Perlglanzagentien, Pflanzenextrakte, Vitamine, Wirkstoffe, Konservierungsmittel, Bakterizide, Farbstoffe, Verdickungsmittel, anfeuchtende und/oder feuchthaltende Substanzen, Fette, Öle, Wachse, sofern nicht ausgeschlossen, oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate enthalten.
Moisturizer können vorteilhaft auch als Antifaltenwirkstoffe zum Schutz vor Hautveränderungen, wie sie z. B. bei der Hautalterung auftreten, verwendet werden.

Nachfolgende Beispiele zeigen bevorzugte Ausführungsformen der erfindungemäßen Formulierungen. Soweit nichts anderes angegeben beziehen sich die Zahlenwerte auf Gewichtsanteile, bezogen auf die Gesamtmasse der Formulierungen.

### UV-Filterhaltige Formeln

| Beispiel Emulsion | **1** | **2** | **3** | **4** | **5** |
|---|---|---|---|---|---|
| Glyceryl Stearat Citrat | 2 | 2 | 2 | | |
| Glyceryl Stearat SE | | | | 1 | 1 |
| Cetearyl Alkohol + PEG-40 Rizinusöl + | | | | 2,5 | 2,5 |
| Natrium Cetearyl Sulfat | | | | | |
| Cetearylalkohol | | | 1,5 | 1 | |
| Stearylalkohol | 2 | 1,5 | | | |
| Acrylates/C₁₀₋₃₀ Alkyl Acrylat Crosspolymer | | 0,2 | | | 0,1 |
| Xanthan Gum | 0,4 | | 0,2 | 0,2 | 0,3 |
| C₁₂₋₁₅ Alkyl Benzoat | | 3 | | | 5 |
| Dicaprylyl Carbonat | | 2 | | | |
| Myristylmyristat | | | 2 | | 1 |
| Butylenglycol Dicaprylat/Dicaprat | | | 3 | | 3 |
| Propylheptyl Caprylat | 5 | | 5 | 2 | |
| Dicaprylyl Ether | | | | | 2 |
| Octyldodecanol | 1 | | | | |
| Cyclisches Silikon | | 5 | 5 | 1 | 10 |
| Dimethicon / PPG-20 Crosspolymer und Isodecyl Neopentanoat | 10 | | | 2,25 | 12,5 |
| Dimethicon / PPG-20 Crosspolymer und Isododecan | | 5 | 10 | | |
| Lineares Silikon | 6 | 5 | | 5 | |
| 1-Methyl-1,3-propandiol | 5 | 8 | | | |
| Glycerin | 3 | 5 | 3 | 5 | 3 |
| Octan-1,2-diol | 2 | | 1 | | |
| 2-Methyl-1,3 propandiol | | | | 2 | |
| Bis-Vinyl Dimethicon / PPG-20 Crosspolymer | | 4 | 1 | 0,25 | |
| 2-(4'-Diethylamino-2'-hydoxybenzoyl)-benzoesäurehexylester | 3 | 5 | 1 | 0,5 | 2 |
| Octocrylen | | | 5 | | |
| Titandioxid | | 0,5 | 1 | | 2 |
| Phenylbenzimidazol Sulfonsäure | | | | 4 | 2 |
| Octylsalicylat | | | 5 | | |
| Polysilicon-15 | | | | | 2 |
| Ethylhexylmethoxycinnamat | | 10 | | | |
| Methylen Bis-Benzotriazolyl | 3 | | | 2 | |
| Tetramethylbutylphenol | | | | | |
| Ethylhexyltriazin | 3 | 2 | | | 3 |
| Bis-Ethylhexylphenol Methoxyphenyl Triazin | | 2 | | | |
| PVP/Hexadecen Copolymer | 0,2 | 0,5 | 0,1 | 0,2 | |
| Vitamin E Acetat | 0,2 | 0,2 | 0,2 | 0,3 | 0,1 |
| Na₂H₂EDTA | 0,1 | 0,1 | 0,2 | 0,2 | 0,2 |
| Parfüm | 0,2 | 0,3 | 0,3 | 0,4 | 0,25 |
| Konservierungsmittel | 0,3 | 0,5 | 1 | 0,4 | 0,6 |
| Natriumhydroxid | q.s. | q.s. | q.s. | q.s. | q.s. |
| Wasser | ad 100,0 | ad 100,0 | ad 100,0 | ad 100,0 | ad 100,0 |

Es bilden sich stabile Emulsionen.

| Beispiel | 6 | 7 | 8 | 9 | 10 | 11 |
|---|---|---|---|---|---|---|
| Glyceryl Stearat SE | 1 | 2 | | | | |
| Cetearyl Alkohol + PEG-40 Rizinusöl+ Natrium Cetearyl Sulfat | 2 | 1,5 | | | | |
| Natrium Stearoylglutamat | | | 0,2 | 0,4 | | |
| Sucrosepolystearat | | | 1 | 0,8 | | |
| Polyglyceryl-3 Methylglycose Distearat | | | | | 3 | 2,5 |
| Sorbitanstearat | | | | | 1 | 0,8 |
| Copolymer aus Vinylpyrrolidon und Acrylsäure | | | 1,5 | | | |
| Stearylalkohol | | | | | 2 | |
| Cetylalkohol | 2 | 2 | | | | 2 |
| Acrylates/C₁₀₋₃₀ Alkyl Acrylat Crosspolymer | | | | 0,3 | 0,2 | 0,1 |
| Carbomer | | | | 0,2 | | 0,1 |
| Xanthan Gum | 0,5 | 0,3 | | | | |
| C₁₂₋₁₅ Alkyl Benzoat | | 3 | 5 | | | |
| Butylenglycol | | | | | | |
| Dicaprylat/Dicaprat | | | | | | |
| Dicaprylyl Carbonat | 4 | | 2 | | 2 | |
| Octyldodecanol | | | | | | 2 |
| Cyclisches Silikon | 3 | | 2 | 10 | 5 | |
| Dimethicon / PPG-20 Crosspolymer und Isodecyl Neopentanoat | 25 | | 18 | 5 | | 4,3 |
| Bis-Vinyl Dimethicon/PPG-20 Crosspolymer und Isododecan | | 12,25 | 10 | 5 | 15 | 3,5 |
| Lineares Silikon | 2 | 5 | 8 | 5 | | |
| Glycerin | 1 | 2 | 4 | 5 | 2 | 8 |
| Ethanol | 4 | 3 | 4 | | | 4 |
| 2-(4'-Diethylamino-2'-hydoxybenzoyl)-benzoesäurehexylester | 2 | | 1,5 | | 0,5 | 6 |
| Octocrylen | | | | | | 7,5 |
| Butyl Methoxydibenzoylmethan | | 2,5 | | 3 | 2 | |
| Ethylhexylmethoxycinnamat | 4 | | | | | 7,5 |
| Octylsalicylat | | | 5 | | | |
| Phenylbenzimidazol Sulfonsäure | 2 | 1 | 1 | | | |
| Titandioxid | | | | 5 | | 3 |
| Bis-Ethylhexylphenol Methoxyphenyl Triazin | | | 2 | | | 2 |
| Isoamylmethoxycinnamat | | 5 | | | | |
| Ethylhexyltriazin | | | | | 2 | 2 |
| Vitamin E Acetat | 0,1 | 0,2 | 0,1 | 0,5 | 0,25 | 0,3 |
| Na₂H₂EDTA | 0,05 | 0,2 | 0,2 | 0,2 | 0,2 | 0,5 |
| Stärke | 2 | 1 | | | 3 | |
| Parfüm | | | 0,2 | 0,1 | 0,3 | 0,25 |
| Konservierungsmittel | q.s. | q.s. | q.s. | q.s. | q.s. | q.s. |
| Natriumhydroxid, Farbstoffe | q.s. | q.s. | q.s. | q.s. | q.s. | q.s. |
| Wasser | ad 100,0 | ad 100,0 | ad 100,0 | ad 100,0 | ad 100,0 | ad 100,0 |

| Beispiel | 12 | 13 | 14 | 15 | 16 | 17 |
|---|---|---|---|---|---|---|
| Glycerylstearat | 2,5 | 2 | 1,2 | 1 | | |
| PEG-40 Stearat | 1 | | | | | |
| PEG-100 Stearat | | 2,5 | | | | |
| Ceteareth-20 | | | | | 1 | |
| Stearinsäure | | | 2,5 | 3 | | |
| Cetearylalkohol | 4 | | | 2 | | |
| Stearylalkohol | | 2 | 1 | | | |
| Cetylalkohol | | | 1 | 1 | | |
| Acrylates/C₁₀₋₃₀ Alkyl Acrylat Crosspolymer | | | 0,05 | 0,2 | 0,2 | 0,5 |
| Carbomer | 0,1 | | 0,2 | | | |
| Xanthan Gum | | 0,3 | | | | |
| Triheptanoin | | | 2 | | | |
| C₁₂₋₁₅ Alkyl Benzoat | | 7 | | | 3 | 3 |
| Butylenglycol | 2 | 4 | | | 5 | |
| Dicaprylat/Dicaprat | | | | | | |
| Dicaprylyl Carbonat | 4 | | | | | 2 |
| Phenylethylbenzoat | | | 5 | 5 | 4 | |
| Diisopropylsebacat | | 3 | | 5 | | |
| Cyclisches Silikon | 3 | | | | | |
| 2-Propylheptyloctanoat | 4 | | 3 | | 2 | 7,5 |
| Bis-Vinyl Dimethicon / PPG-20 Crosspolymer und Isodecyl Neopentanoat | 20 | 2,5 | | 12,5 | | 10 |
| Dimethicon / PPG-20 Crosspolymer und Isododecan | | 7,5 | 6 | | 5 | |
| Glycerin | 7,5 | 5 | 4 | 3 | 5 | 2 |
| Ethanol | | | 2 | | 4 | 2 |
| Bis-Ethylhexyloxyphenol Methoxyphenyltriazin | 2 | 3 | 0,5 | 1,5 | 3 | 2 |
| Ethylhexylmethoxycinnamat | | | 7 | | 8 | 1 |
| Octylsalicylat | | | | | 5 | 3 |
| Homosalat | | | 3 | | | |
| Octocrylen | 5 | | | 4 | | 4 |
| Phenylbenzimidazol Sulfonsäure | 1 | | | 2 | | |
| Butyl Methoxydibenzoylmethan | 2 | | 3 | 4 | 1 | |
| 2,4,6-Tris-(biphenyl)-1,3,5-triazin | | 2 | | | 1 | |
| Diethylhexylbutamidotriazin | | | 2 | 1,5 | | 2 |
| Ethylhexyltriazin | | 1 | | 1,5 | | |
| Tapiokastärke | 1 | | 2,5 | | | 0,5 |
| Natrium-Stärke Octenylsuccinat | | | | 1 | | |
| Creatin | 0,5 | | | 0,5 | 0,2 | |
| Coenzym Q 10 | 0,1 | 0,02 | | | | |
| Vitamin E Acetat | 0,5 | | 0,3 | 0,3 | 0,8 | 0,5 |
| Na₂H₂EDTA | 0,1 | | | | | 0,5 |
| Parfüm | 0,2 | 0,3 | 0,4 | 0,5 | | |
| Parabene | 0,5 | 0,4 | 0,5 | 0,3 | 0,3 | 0,4 |
| Natriumhydroxid, Farbstoffe | q.s. | q.s. | q.s. | q.s. | q.s. | q.s. |
| Wasser | ad 100,0 | ad 100,0 | ad 100,0 | ad 100,0 | ad 100,0 | ad 100,0 |

| W/O-Emulsionen | 17 | 18 | 19 | 20 | 21 |
|---|---|---|---|---|---|
| Triglycerindiisostearat | 1,0 | 0,5 | 0,25 | 2,0 | 3,0 |
| Diglycerindipolyhydroxystearat | 1,0 | 1,5 | 1,75 | 3,0 | 2,0 |
| Paraffinöl | 12,5 | 10,0 | 8,0 | 5,0 | 11,5 |
| Vaseline | 8,0 | 6,0 | 5,0 | 12,0 | 2,5 |
| Hydrierte Kokosglyceride | 2,0 | 1,0 | 2,5 | 5,0 | 0,25 |
| Decyloleat | 0,5 | 0,75 | 1,0 | 2,0 | 0,25 |
| Octyldodecanol | 0,5 | 1,0 | 0,75 | 3,0 | 0,25 |
| Aluminiumstearat | 0,4 | 0,3 | 0,6 | 1,0 | 0,05 |
| Dicaprylylcarbonat | 0,1 | 0,05 | 0,15 | 0,5 | 1,0 |
| Hydriertes Rizinusöl | 0,5 | 0,75 | 1,0 | 2,5 | 5,0 |
| Dimethicon / PPG-20 Crosspolymer und Isododecan | --- | 12,5 | 2,5 | --- | --- |
| Bis-Vinyl Dimethicon / PPG-20 Crosspolymer und Isodecyl Neopentanoat | 7,5 | --- | --- | 5 | 20 |
| Magnesiumsulfat | 0,5 | 0,6 | 0,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 0,2 | 0.05 | 0.4 | 0.3 | 2,0 |
| Parfum | q,s, | q,s, | q,s, | q,s, | q,s, |
| Ethanol | 2,0 | --- | 5,0 | --- | --- |
| Capryl-/Caprinsäuretriglycerid | 2,0 | 2,5 | 3,0 | 5,0 | 0,5 |
| Kaliumsorbat | 0,04 | 0,15 | 0,05 | 0,03 | 0,4 |
| Benzylalkohol | 0,3 | 0,4 | 0,25 | 0,15 | --- |
| Wasser | ad 100 | ad 100 | ad 100 | ad 100 | ad 100 |

| W/O-Emulsionen | 22 | 23 | 24 | 25 | 26 |
|---|---|---|---|---|---|
| PEG-30 Dipolyhydroxystearat | --- | 0,5 | 0,25 | --- | 3,0 |
| Polyglyceryl-2 Dipolyhydroxystearat | 2 | | | | |
| Lanolin Alcohol | | 1,5 | 1,75 | 3,0 | --- |
| Paraffinöl | 8 | 10,0 | 8,0 | 5,0 | 10,5 |
| Vaseline | | 6,0 | 5,0 | 12,0 | |
| Hydrierte Kokosglyceride | 2,0 | 1,0 | 2,5 | 5,0 | |
| Hydriertes Polyisobuten | 0,5 | 0,75 | 1,0 | 2,0 | |
| Octyldodecanol | | 1,0 | | 3,0 | |
| Aluminiumstearat | 0,4 | 0,3 | 0,6 | 1,0 | 0,05 |
| Dicaprylylcarbonat | 0,1 | 0,05 | 0,15 | 0,5 | 4 |
| Hydriertes Rizinusöl | | 0,75 | 1,0 | 2,5 | |
| Microcrystalline Cellulose | | 1,0 | 0,75 | 0,25 | |
| Magnesiumsulfat | 0,2 | 0,5 | 0,5 | 0,3 | 0,6 |
| Glycerin | 3,0 | 5,0 | 10,0 | 15,0 | 1,5 |
| Zitronensäure | 0,2 | 0,1 | 0,2 | 0,3 | 1,0 |
| Parfum | q,s, | q,s, | q,s, | q,s, | q,s, |
| 1,3 Butylenglykol | 2,0 | --- | 5,0 | --- | --- |
| Lineares Silikon | 2 | 5 | | | 4 |
| Natrium Stärke Octenylsuccinat | | 1 | 2 | 0,5 | |
| Titandioxid | 5 | | | | 3 |
| Polysilicon-15 | | 4 | 3 | | |
| Butyl Methoxydibenzoylmethan | | 2 | | | 4 |
| Octocrylen | | 4 | | | 9 |
| Ethylhexylcinnamat | 3 | | | | |
| Dimethicon / PPG-20 Crosspolymer und | --- | 15,0 | --- | 3,0 | --- |
| Isodecyl Neopentanoat | | | | | |
| Bis-Vinyl Dimethicon / PPG-20 | 5,0 | --- | 7,5 | --- | 17,5 |
| Crosspolymer und Isododecan | | | | | --- |
| Kaliumsorbat | 0,3 | 0,4 | 0,25 | 0,15 | 0,1 |
| Talkum | --- | --- | 0,05 | --- | 0,1 |
| Wasser | ad 100 | ad 100 | ad 100 | ad 100 | ad 100 |

| W/S-Emulsion | 27 | 28 | 29 | 30 | 31 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclisches Silikonöl (Cyclomethicon) | 12,5 | 15 | 22,0 | 20,0 | 15,5 |
| Lineares Silikonöl (Dimethicon) | 5,0 | 13,0 | 5,0 | 12,0 | 15,0 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Zitronensäure | 0,2 | 0,1 | 0,2 | 0,3 | 1,0 |
| Natriumcitrat | 1,0 | 0,1 | 0,4 | 0,9 | 2,5 |
| Parfum | q,s, | q,s, | q,s, | q,s, | q,s, |
| Kaliumsorbat | 0,4 | 0,1 | 0,05 | 0,3 | 0,4 |
| Microcrystalline Cellulose | 1,0 | 0,1 | 0,5 | 0,25 | 0,1 |
| 2,4,6-Tris-(biphenyl)-1,3,5-Triazin | 2 | | | | |
| Octylsalicylat | 5 | | | | |
| Dimethicon / PPG-20 Crosspolymer und | 2,5 | 12,5 | --- | --- | --- |
| Isodecyl Neopentanoat | | | | | |
| Bis-Vinyl Dimethicon / PPG-20 | --- | --- | 10 | 7,5 | 5,0 |
| Crosspolymer und Isododecan | | | | | |
| Benzylalkohol | --- | --- | 0,05 | --- | 0,1 |
| Modifizierte Stärke | --- | 2,5 | --- | 0,15 | - |
| Wasser | ad 100 | ad 100 | ad 100 | ad 100 | ad 100 |

| W/S-Emulsionen | 32 | 33 | 34 | 35 | 36 |
|---|---|---|---|---|---|
| Cetyl PEG/PPG-10/1 Dimethicone | 1,0 | --- | -- | 3,0 | 5,0 |
| Cylomethicon + PEG/PPG-18/18 Dimethicon (90:10) | 10,0 | 12,5 | 25 | -- | -- |
| Cyclisches Silikonöl (Cyclomethicon) | 12,5 | 15 | 8,0 | 20,0 | 17,5 |
| Lineares Silikonöl (Dimethicon) | 5,0 | 13,0 | 5,0 | 12,0 | 15,0 |
| Hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Natriumchlorid | 2,0 | 0,6 | 2,5 | 0,7 | 1,0 |
| Glycerin | 3,0 | 5,0 | 10,0 | 15,0 | 1,5 |
| Milchsäure | 0,2 | 0,1 | 0,2 | --- | --- |
| Natriumlactat | 0,2 | 1,0 | 0.05 | --- | --- |
| Parfum | q,s, | q,s, | q,s, | q,s, | q,s, |
| Microcrystalline Cellulose | 1,0 | 0,1 | 1,5 | 2,5 | 0,1 |
| Kaliumsorbat | 0,3 | 0,4 | 0,25 | 0,15 | --- |
| Stearyldimethicon | 0,5 | --- | 0,7 | --- | --- |
| Dimethicon / PPG-20 Crosspolymer und Isododecan | 1,5 | | | 15,0 | |
| Bis-Vinyl Dimethicon / PPG-20 | | 2,25 | 3,0 | | 1,0 |
| Crosspolymer und Isodecyl Neopentanoat | | | | | |
| Modifizierte Stärke | --- | 2,5 | --- | 0,15 | - |
| Wasser | ad 100 | ad 100 | ad 100 | ad 100 | ad 100 |

| Si/ W-Emulsion | 37 | 38 | 39 | 40 | 41 |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| cyclisches Silikonöl (Cyclomethicon) | 12,5 | 15 | 25,0 | 10,0 | 7,5 |
| lineares Silikonöl (Dimethicon) | 5,0 | 15,0 | 5,0 | 12,0 | 15,0 |
| Dimethicon / PPG-20 Crosspolymer und Isodecyl Neopentanoat | --- | 0,75 | 10,0 | --- | --- |
| Bis-Vinyl Dimethicon / PPG-20 Crosspolymer und Isododecan | 0,5 | --- | --- | 2,0 | 25,5 |
| Octyldodecanol | 0,5 | 1,0 | 0,75 | 3,0 | 0,25 |
| Glycerin | 5,0 | 7,5 | 10,0 | 3,0 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Ethylhexylcinnamat | | | | | 5 |
| Bis-Ethylhexyloxyphenol Methoxy- | | | | | 1 |
| phenyltriazin | | | | | |
| Modifizierte Stärke | --- | 2,5 | --- | 0,15 | - |
| Wasser | ad 100 | ad 100 | ad 100 | ad 100 | ad 100 |

| Hydrodispersionsgel | 42 | 43 | 44 | 45 | 46 |
|---|---|---|---|---|---|
| Silikonöl, cyclisch | 8 | 10 | 5 | 3 | --- |
| Silikonöl, linear | --- | --- | --- | --- | 3 |
| Dimethiconol | 1 | 2 | 3 | --- | 3 |
| Ethanol | 1,0 | 5,0 | 7,5 | 1,5 | 3,0 |
| Natriumpolyacrylat | 0,2 | 0,3 | 0,3 | 0,4 | 0,10 |
| Methylpropandiol | 2 | 3 | 4 | 5 | --- |
| Glycerin | 9 | 15 | 5 | 7,5 | 25 |
| Carbomer | 0,2 | 0,3 | 0,2 | 0,4 | 0,15 |
| Acrylates/C10-30Alkyl Acrylate Crosspolymer | 0,2 | 0,15 | 0,3 | 0,4 | 0,10 |
| Carrageenan (Chondrus Crispus) | - | --- | --- | --- | 2 |
| Hamamelis Extrakt | 0,1 | 0,2 | 0,3 | 0,4 | --- |
| Parfum | q.s. | q.s. | q.s. | q.s. | q.s. |
| Polysilicin-15 | 3 | | | | |
| Phenylbenzimidazol Sulfonsäure | 1 | | | | |
| Dimethicon / PPG-20 Crosspolymer und Isodecyl Neopentanoat | --- | 1,5 | --- | --- | 8,5 |
| Bis-Vinyl Dimethicon / PPG-20 Crosspolymer und Isododecan | 0,5 | --- | 5,0 | 3,5 | --- |
| Wasser | ad 100 | ad 100 | ad 100 | ad 100 | ad 100 |

| Lippenstifte | Anteil in Gew.-% | | | |
|---|---|---|---|---|
| | 47 | 48 | 49 | 50 |
| Rizinusöl | ad 100 | | 6 | 3 |
| Caprylic-/Capric Triglyceride | 20 | 20 | ad 100 | 3 |
| Pentaerythrityl Tetraisostearat | | 5 | | |
| Macadamiaöl | | | 2 | |
| Octyldodecanol | 20 | 5 | 6 | 6 |
| Hydriertes Polyisobuten | | | 10 | 1 |
| (z.B. Parleam Type 4, Rossow | | | | |
| Cosmétiques) | | | | |
| Polyisobuten | | 2 | 3 | |
| Isopropylpalmitat | | 3,5 | 6 | 2 |
| Jojobaöl | 6 | | | 1 |
| Lanolinöl | 5 | | 9 | 1 |
| PEG-45/ Dodecyl Glykol Copolymer | | | | 2 |
| Polyglyceryl-3 Diisostearat | 3,7 | 3,5 | | 2,4 |
| Bis-Diglyceryl Polyacyladipat-2 | | 6 | | |
| Cetearyl Alkohol | 6 | 0,5 | | |
| Cetylpalmitat | 7,5 | | | 1 |
| C20-40 Alkyl Stearat | 16 | | 3 | 8 |
| Dimethicon / PPG-20 Crosspolymer und Isodecyl Neopentanoat | 10 | | 20 | |
| Bis-Vinyl Dimethicon / PPG-20 Crosspolymer und Isododecan | | 8 | | 5 |
| Carnaubawachs | | 5 | 4 | 2 |
| Candelillawachs | | 3 | 4 | |
| Bienenwachs | 6 | 2 | | |
| Mikrokristallines Wachs | | 8 | 4 | 8 |
| PVP/Eicosencopolymer | | | 0,5 | 0,2 |
| 4-Methylbenzyliden Campher | | | | 5 |
| Octyl Methoxycinnamat | 3 | 3 | 3 | 2,5 |
| Ethylhexyl Triazon | | | 2 | 6 |
| Octocrylen | | 3 | | |
| Butyl Methoxydibenzoylmethan | 2 | | | 0,8 |
| Lauroyl Lysin | 0,5 | | 0,6 | |
| Red 7 Lake | 0,5 | 2,6 | 4,2 | 6 |
| Titandioxid | | 0,6 | 1 | 2 |
| Eisenoxide | | 2,4 | 2 | 1 |
| Effektpigmente | | 6 | 3,5 | |
| Glimmer | | | 4 | |
| Diammonium Citrat | 0,08 | | | |
| Zitronensäure | 0,05 | | | |
| Glycerin | | | | 10 |
| Tocopheryl Acetate | 0,5 | | 0,5 | 1 |
| Ascorbyl Palmitate | | 0,2 | 0,3 | |
| Ubichinon | | 0,5 | | |
| Parfum, Konservierungsmittel, BHT, Neutralisationsmittel, Sequestriermittel | q. s. | q. s. | q. s. | q. s. |
| Wasser | 2,5 | | | ad 100 |

| | | |
|---|---|---|
| hochglänzende Lippenstifte | | 51 |
| | Dimethicone /PPG-20 Crosspolymer und Isodecyl Neopentanoate | 10 |
| | Synthetisches Wachs | 5 |
| | Mikrokristallines Wachs | 5 |
| | Candelillawachs | 5 |
| | Shea Butter | 3 |
| | Myristyl Lactat | 7 |
| | Polyisobuten | 15 |
| | Ricinusöl | 19,6 |
| | Octyldodecanol | 10 |
| Phase A | Pentaerythrityl Tetraisostearat | 15 |
| | Red 7 Lake | 4 |
| Phase B | Propylparaben | 0,2 |
| | Tocopheryl Acetat | 1 |
| | Parfüm | 0,2 |
| | | 100 |
| | | |
| | Lippenstift | 52 |
| | | |
| | Bis-Vinyl Dimethicon / PPG-20 Crosspolymer und Isodecyl Neopentanoat | 5 |
| | Synthetisches Wachs | 3 |
| | Mikrokristallines Wachs | 3 |
| | Candelillawachs | 6 |
| | Shea Butter | 3 |
| | Myristyl Lactat | 7 |
| | Polyisobuten | 25 |
| | Ricinusöl | 14,1 |
| | Octyldodecanol | 10 |
| Phase A | Pentaerythrityl Tetraisostearate | 15 |
| Phase B | Red 7 Lake | 4 |
| | Propylparaben | 0,2 |
| | Butyl Methoxybenzoylmethane | 0,5 |
| | Octyl Methoxycinnamate | 3 |
| | Tocopheryl Acetate | 1 |
| | Parfüm | 0,2 |
| | | 100 |
| | | |
| Phase A | Lippenstift | |
| | | 54 |
| | Isododecan Dimethicone /PPG-20 Crosspolymer | 5 |
| | Bis-Vinyl Dimethicone / PPG-20 Crosspolymer und Isodecyl Neopentanoat | 2 |
| | Synthetisches Wachs | 3 |
| | Mikrokristallines Wachs | 3 |
| | Candelillawachs | 6 |
| | Shea Butter | 3 |
| | Myristyl Lactate | 7 |
| | Polyisobuten | 16,2 |
| | Ricinusöl | 14,3 |
| | Octyldodecanol | 15 |
| | Pentaerythrityl Tetraisostearate | 8 |
| | Red 7 Lake | 3 |
| | Propylparaben | 0,2 |
| | Avocadoöl | 5 |
| | Sucrose Acetat Isobutyrat | 3 |
| Phase B | Lanolinöl | 5 |
| | Tocopheryl Acetat | 1 |
| | Parfüm | 0,3 |
| | | 100 |
| | | |
| | | |
| Phase A | Lippenstift | |
| | | 55 |
| | Isodecyl Neopentanoat Dimethicone /PPG-20 Crosspolymer | 3 |
| | Bis-Vinyl Dimethicon / PPG-20 | 7 |
| | Crosspolymer und Isodecyl Neopentanoat | |
| | Synthetisches Wachs | 2 |
| | Mikrokristallines Wachs | 3 |
| | Candelillawachs | 7 |
| | Shea Butter | 3 |
| | Myristyl Lactat | 7 |
| | Polyisobuten | 11,5 |
| Phase B | Ricinusöl | 14 |
| | Octyldodecanol | 15 |
| | Pentaerythrityl Tetraisostearat | 15 |
| | Red 7 Lake | 3 |
| | Propylparaben | 0,2 |
| | Sucrose Acetate Isobutyrat | 3 |
| | Lanolinöl | 5 |
| | Tocopheryl Acetat | 1 |
| | Parfüm | 0,3 |
| | | 100 |

Die Phasen A und B werden homogen vermischt..

| transfer resistente Lippenstifte | 56 | 57 | 58 | 59 | 60 | 61 |
|---|---|---|---|---|---|---|
| Dimethicone /PPG-20 Crosspolymer und Isodecyl Neopentanoat | 5 | | 10 | | 3 | |
| Bis-Vinyl Dimethicon / PPG-20 Crosspolymer und Isododecan | | 5 | | 2 | 2 | 7 |
| Synthetisches Wachs | 11 | 10 | 12 | 15 | 10 | 12 |
| Schellack Wachs | 2,5 | | 1,5 | 2 | 5 | |
| Sonnenblumenwachs | | 4 | | | | 3 |
| Isoparaffin C₁₀₋₁₂ | 50 | 40 | 50 | 55 | 50 | 60 |
| Pigmente | 10 | 10 | 10 | 10 | 10 | 10 |
| Trimethylsiloxysilicat | 10 | 8 | 5 | 12 | 6 | 5 |
| Sucrose Acetate Ester | | 2 | | | 2 | |
| PVP/Hexadecen Copolymer | 2 | | | 3 | | |
| PVP/Eicosen Copolymer | | | 1 | | | 1 |
| Propylparaben | 0,1 | 0,1 | 0,1 | 0,1 | 0,1 | 0,1 |
| BHT | 0,03 | 0,03 | 0,03 | 0,03 | 0,03 | 0,03 |
| Polyisobuten | add to 100 | | | | add to 100 | |
| Hydriertes Polydecen | | add to 100 | | | | add to 100 |
| Hydriertes Polyisobuten | | | | add to 100 | | |
| Jojobaöl | | | add to 100 | | | |

| Emulsionsmascara | 62 | 63 | 64 | 65 | 66 |
|---|---|---|---|---|---|
| | Gew.-% | Gew.-% | Gew.-% | Gew.-% | Gew.-% |
| Stearinsäure | 3,50 | 4,00 | 4,00 | 4,00 | 3,50 |
| Ozokerit | 7,00 | 6,00 | 5,00 | 5,00 | 7,00 |
| Carnaubawachs | 3,00 | 3,50 | 3,50 | 3,50 | 3,00 |
| Neutralöl | 3,00 | 4,00 | - | 1,00 | 3,00 |
| Oleylalkohol | - | - | 3,00 | - | - |
| Sorbitansesquioleat | 1,50 | 1,80 | 1,80 | 1,50 | 1,50 |
| Triethanolamin | 1,00 | 1,10 | 1,10 | 1,10 | 1,00 |
| Lycra ® | 0,05 | 0,50 | 0,75 | 1,00 | 0,50 |
| PVP Copolymer | - | - | - | - | 3,00 |
| Butandiol | 2,00 | 1,50 | 1,40 | 1,50 | 2,00 |
| Talkum | 5,00 | 3,00 | - | 2,00 | 5,00 |
| Bis-Vinyl Dimethicon / PPG-20 Crosspolymer und Isododecan | 2 | | | 4,5 | |
| Dimethicone /PPG-20 Crosspolymer und Isodecyl eopentanoat | | 5 | 10 | | 20 |
| Farbpigmente (Eisenoxide) | 8,00 | 9,00 | 10,00 | 9,00 | 8,00 |
| Calciumpanthotenat | 1,30 | 4,80 | 0,10 | 0,50 | 1,30 |
| Gamma-Oryzanol | 2,30 | 0,20 | 5,00 | 2,00 | 2,30 |
| Konservierungsmittel | q.s. | q.s. | q.s. | q.s. | q.s. |
| Wasser, demineralisiert | ad 100,00 | ad 100,00 | ad 100,00 | ad 100,00 | ad 100,00 |

| Wasserfeste Mascara | 67 | 68 | 69 |
|---|---|---|---|
| | Gew.-% | Gew.-% | Gew.-% |
| Quaternium-18-Hectorit | 10,00 | 12,00 | 10,00 |
| Talkum | 5,00 | 4,00 | 5,00 |
| Ethanol | 2,50 | 2,75 | 2,50 |
| Lycra ® | 0,05 | 0,40 | 1,00 |
| Carnaubawachs | 2,00 | - | 3,00 |
| PVP/Eicosencopolymer | 20,00 | 18,00 | 17,00 |
| Perlglanzpigmente | 5,00 | 3,00 | 3,00 |
| Farbpigmente(Eisenoxide) | 10,00 | 10,00 | 10,00 |
| Nylon-6 | - | 2,00 | 1,00 |
| Cyclomethicon | 5,00 | - | 3,00 |
| Bis-Vinyl Dimethicon / PPG-20 Crosspolymer und Isodecy Neopentanoat | 2 | | 7 |
| Isododecan und Dimethicone /PPG-20 Crosspolymer | | 10 | |
| Konservierungsmittel | q.s. | q.s. | q.s. |
| Calciumpanthotenat | 0,80 | 0,10 | 0,20 |
| Gamma-Oryzanol | 0,20 | 5,00 | 1,00 |
| Isoparaffin C₁₀₋₁₂ | ad 100,00 | ad 100,00 | ad 100,00 |

| Foundations | 70 | 71 | 72 | 73 |
|---|---|---|---|---|
| | | | | |
| Wasser | Ad 100 | Ad 100 | Ad 100 | Ad 100 |
| Glycerin | 7 | 5 | 5 | 10 |
| Cyclomethicon + PEG/PPG-19/19 Dimethicon | | 4 | 2 | |
| Cetyl PEG/PPG-10/1 Dimethicon | 4 | | | 5 |
| Propylene Carbonat | 0,05 | 0,06 | 0,06 | 0,03 |
| Disteardimonium Hectorit | 0,25 | 0,4 | 0,2 | 0,1 |
| Natriumchlorid | 2 | | 1 | 2 |
| Squalan | 0,5 | | 0,5 | |
| Dicaprylyl Carbonat | | 5 | | 5 |
| Cyclomethicon | 10 | 12 | 20 | 15 |
| Dicaprylyl Ether | 2 | | | |
| Caprylic/Capric Triglycerid | 2 | | | |
| Dimethicon | 2 | 3 | | 4 |
| Bis-Vinyl Dimethicon / PPG-20 Crosspolymer und Isodecyl Neopentanoat | 3 | | 10 | 2 |
| Isododecan und Dimethicone /PPG-20 Crosspolymer | | 15 | | 8 |
| Myristyl Laktat | | | 1 | |
| Lecithin | 1 | | 0,5 | |
| Dimethicon + Trimethylsiloxysilikat | | | 3,5 | 2 |
| Lauroyl Lysin | 5 | | 2,5 | 3 |
| Talkum | 2 | 3 | 0,5 | |
| Nylon 6/12 | | 3 | 2 | 4 |
| Silika | | 3 | 4 | 3 |
| Dimethicon + Dimethicon Crosspolymer | 6 | 4 | | |
| Methyl Methacrylat Crosspolymer | | | 1 | 2 |
| Polymethylsilsesquioxan | 2 | 1 | | |
| VP/VA Copolymer | 0,1 | | | 0,2 |
| PVP/Hexadecen Copolymer | 0,1 | | 0,5 | |
| Titandioxid (CI 77891) | 6 | 3 | 3 | 8 |
| Farbpigmente (Cl 77492 + Cl 77491 + Cl 77499) | 5 | 5 | 2 | 7 |
| Effektpigmente (z.B. beschichtetes Mica) | | 3 | 1 | |
| Titandioxid | 1 | 2 | | |
| Dimethicodiethylbenzylmalonat (Polysilicone-15) | 0,5 | 2 | 4 | 2,5 |
| 2-Phenylbenzimidazol-5-sulfonsäuresalze | 1 | 5 | 2 | 3 |
| Sodium Ascorbyl Phosphat | | | 0,1 | 0,2 |
| Tocopheryl Acetat | 0,5 | 0,5 | 1 | 1,5 |
| Ubiquinon | | 0,05 | | |
| Phenoxyethanol + Parabene | 1 | 0,7 | | |
| Tetrasodium Iminodisuccinat | | 0,2 | | |
| Diazolidinyl Urea | | | 0,3 | 0,3 |
| Parfum | 0,5 | 0,5 | 0,5 | 0,5 |

### Deo / AT- Formulierungen

| Stifte | 74 | 75 | 76 | 77 | 78 | 79 |
|---|---|---|---|---|---|---|
| Aluminiumchlorhydrat | 20 | | | | | |
| Aluminium Zirkonium | | 20 | 16 | 16 | 16 | 16 |
| Tetrachlorhydrex Glycin | | | | | | |
| Stearylalkohol | 20 | 20 | 20 | 18 | 18 | 18 |
| Hydriertes Ricinusöl | 1 | 1 | 1 | 1,5 | 1,5 | 1,5 |
| Talkum | 2 | 2 | 4 | 4 | 4 | 4 |
| PPG-14 Butylether | 10 | | | | 5 | |
| C12-15 Alkylbenzoat | | 10 | 5 | | | |
| Capric / Caprylic Triglycerid | | | | 15 | 10 | 5 |
| Bis-Vinyl Dimethicon / PPG-20 Crosspolymer und Isodecyl eopentanoat | 10 | | | | 10 | |
| Dimethicone und Dimethicone /PPG-20 Crosspolymer | | 10 | 10 | 5 | | 10 |
| Cyclisches Silikon | 37 | 37 | 44 | 40,5 | 35,5 | 45,5 |

| Soft Solids | 80 | 81 | 82 | 83 |
|---|---|---|---|---|
| Aluminium Zirkonium Tetrachlorhydrex Glycin | 18 | 18 | 20 | 20 |
| PPG-14 Butylether | 5 | 5 | | |
| Hydriertes Polydecen | | | 9 | 14 |
| Butylstearat | | 5 | 9 | 9 |
| Lineares Silikon | | | 5 | 5 |
| Stearyl Beeswax + Behenyl Beeswax | 8 | 7 | | |
| Hydroxyoctacosanyl Hydroxystearat | | | 7 | 7 |
| Bis-Vinyl Dimethicon / PPG-20 Crosspolymer und Isodecyl Neopentanoat | 10 | | 10 | |
| Dimethicone und Dimethicone /PPG-20 Crosspolymer | | 5 | | 5 |
| Silika | 1 | 1 | 2 | 2 |
| Tapioca Stärke | 5 | 5 | 5 | 5 |
| Myristylmyristat | 3 | | | |
| Glycerylstearat | 2 | 2 | 1 | 1 |
| Cyclisches Silikon | 48 | 52 | 32 | 32 |

| Aerosole | 84 | 85 | 86 | 87 | 88 | 89 |
|---|---|---|---|---|---|---|
| Aluminiumchlorhydrat | 5 | 5 | 6 | 6 | 5 | 5 |
| Disteardimonium Hectorit | 1 | 1 | 0,8 | 0,8 | | |
| Lineares Silikon | 1 | 1 | | | 1 | 1 |
| Capric / Caprylic Triglycerid | 1 | 1 | 1 | 1 | | |
| C12-15 Alkylbenzoat | | | 1 | 1 | 1 | 1 |
| Bis-Vinyl Dimethicon / PPG-20 Crosspolymer und Isododecan | 3,5 | | 4 | | 3 | |
| Dimethicone und Dimethicone /PPG-20 Crosspolymer | | 2 | | 4 | | 3 |
| Cyclisches Silikon | 3,5 | 5 | 7,2 | 7,2 | 5 | 5 |
| Propan/Butan/Isobutan | 85 | 85 | 80 | 80 | 85 | 85 |

Alle aufgeführten Beispiele belegen jeweils die angeführten Verbesserungen hinsichtlich Stabilität, Dispergierbarkeit partikulärer Stoffe (Vermeidung des Absetzens), verbesserter Lichtschutzleistung, verbessertes Hautgefühl und bessere Wirkstofffreisetzung.

## Patentansprüche

1. Kosmetische Formulierung umfassend ein oder mehrere ABC-Siloxanelastomere, erhältlich aus der Reaktion von
A - Organohydrogensiloxanen mit mindestens zwei SiH tragenden Cyclosiloxanringen pro Molekül, mit
B - Stoffen oder Mischungen mit mindestens zwei aliphatischen ungesättigten Gruppen oder Hydrocarbongruppen pro Molekül und einem
C - Hydrosilylationskatalysator,
und ein oder mehrere partikuläre Stoffe.

2. Formulierung nach Anspruch 1, **dadurch gekennzeichnet, dass** als ABC-Siloxanelastomer Dimethicone/PPG-20 Crosspolymer, Dimethicone/ Bis-Isobutyl PPG-20 Crosspolymer und/oder Bis-Vinyl Dimethicone/PPG-20 Crosspolymer gewählt wird.

3. Formulierung nach Anspruch 1 oder 2, **dadurch gekennzeichnet, dass** die partikulären Stoffe gewählt werden aus der Gruppe umfassend UV-Filter, farbgebende Pigmente, Wirkstoffe und/oder Füllstoffe.

4. Formulierung nach Anspruch 1, 2 oder 3, **dadurch gekennzeichnet, dass** der Zubereitung zusätzlich ein oder mehrere polare Öle oder Wachse zugesetzt sind.

5. Formulierung nach Anspruch 4, **dadurch gekennzeichnet, dass** die polaren Öle gewählt werden aus der Gruppe der Öle mit einer Grenzflächenspannung gegen Wasser kleiner 30 mN/m.

6. Formulierung nach einem der vorstehenden Ansprüche, **dadurch gekennzeichnet, dass** keine unpolaren und/oder flüchtigen Öle enthalten sind.

7. Formulierung nach einem der vorstehenden Ansprüche, **dadurch gekennzeichnet, dass** die Formulierung Silikonölfrei ist.

8. Verwendung von ein oder mehreren ABC-Siloxanelastomere, erhältlich aus der Reaktion von
A - Organohydrogensiloxanen mit mindestens zwei SiH tragenden Cyclosiloxanringen pro Molekül, mit
B - Stoffen oder Mischungen mit mindestens zwei aliphatischen ungesättigten Gruppen oder Hydrocarbongruppen pro Molekül und einem
C - Hydrosilylationskatalysator,
zur verbesserten Dispergierbarkeit und/oder zu einem verbessertem Sedimentationsverhalten partikulärer Stoffe in kosmetischen Formulierungen.

9. Verwendung von ein oder mehreren ABC-Siloxanelastomere, erhältlich aus der Reaktion von
A - Organohydrogensiloxanen mit mindestens zwei SiH tragenden Cyclosiloxanringen pro Molekül, mit
B - Stoffen oder Mischungen mit mindestens zwei aliphatischen ungesättigten Gruppen oder Hydrocarbongruppen pro Molekül und einem
C - Hydrosilylationskatalysator,
in kosmetischen Sonnenschutzformulierungen mit einem Gehalt an partikulären UV-Filtern zur Steigerung des UV Schutzes, Verbesserung des sensorischen Hautgefühls und/oder Erhöhung der Viskosität der Formulierung.
